(19) Europäisches Patentamt
European Patent Office
Office européen des brevets

(11) Publication number: **0 519 070 A1**

(12) **EUROPEAN PATENT APPLICATION published in accordance with Art. 158(3) EPC**

(21) Application number: **91903283.9**

(22) Date of filing: **08.02.91**

(86) International application number: **PCT/JP91/00157**

(87) International publication number: **WO 91/13171 (05.09.91 91/21)**

(51) Int. Cl.[5]: **C12Q 1/68**, C12Q 1/04, G01N 33/80, //C12R1:00

(30) Priority: **23.02.90 JP 43814/90**

(43) Date of publication of application: **23.12.92 Bulletin 92/52**

(84) Designated Contracting States: **AT BE CH DE DK ES FR GB GR IT LI LU NL SE**

(71) Applicant: **Chugai Seiyaku Kabushiki Kaisha**
**5-1, 5-chome, Ukima Kita-ku**
**Tokyo115(JP)**

(72) Inventor: **MATSUBARA, Koichi**
**1-36-815, Seishincho 1-chome**
**Edogawa-ku, Tokyo 134(JP)**

(74) Representative: **Vossius & Partner**
**Siebertstrasse 4 P.O. Box 86 07 67**
**W-8000 München 86(DE)**

(54) **METHOD OF JUDGING TYPE OF HUMAN LEUKOCYTE ANTIGEN.**

(57) A method of judging the type of a human leukocyte antigen (HLA) which is a human histocompatible antigen playing an important role in tissue transplantation and diagnosis of autoimmune diseases and drug sensitivity, said method comprising hybridizing a probe labeled with an acridinium ester through a linker arm under hybridization conditions to the product of amplification of either cDNA prepared from mRNA contained in the specimen or genomic DNA contained in the specimen; decomposing selectively the labeled portion of the unhybridized probe and that of the probe which is hybridized but has mismatching of one or more bases in the crossing; and determining the residual chemiluminescence. This method makes it possible to judge the subtype of the class II gene of HLA, which has been serologically impossible heretofore.

Fig. 9

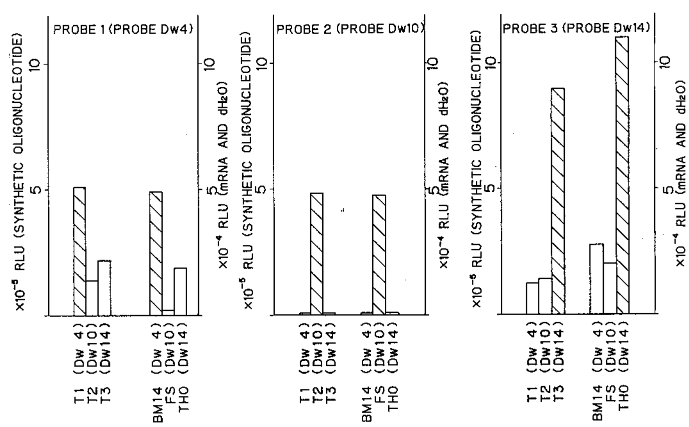


EP 0 519 070 A1

## BACKGROUND ART

This invention relates to a method of identifying various types of human leukocyte antigens (HLA) which are human major histocompatibility complexes that are important to organ transplantation or diagnosis for autoimmune diseases or drug sensitivity.

Identification of HLA types is conventionally performed by serological methods as regards antigens A, B and C of class I and fairly precise type identification has become possible due to improvements in anti-sera [B. Dupoun et al., Tenth International Histocompatability Workshop Newsletter, 2 (1987)]. On the other hand, the differences between antigens of class II are so small that it has been almost impossible to achieve precise type identification by serological methods.

A method of gene amplification (or "polymerase chain reaction" which is hereunder abbreviated as PCR) has been recently invented [R.K. Saiki et al., Science, 239, 487 - 491 (1988)] and this makes it possible to amplify a specific genomic DNA. Under the circumstances, attempts are being made to identify antigen types of class II at the molecular level. In one of these approaches, a region containing the hypervariable area of a gene of class II is amplified and evaluated by dot blotting with a $^{32}P$ labelled probe or specific digestion with restriction enzymes [see "Tanpaku, Kakusan, Koso (Proteins, Nucleic Acids and Enzymes)", 34, 1989 - 2002 (1989), and Human Immunology, 21, 239 - 248 (1989)].

Identifying antigen types of class II is fairly difficult by serological methods and must be performed at the molecular level. Following the development of the PCR method which enabled convenient amplification of a specific gene, many researchers have been engaged in the field of interest. However, the ability of the dot blotting method is not high enough to detect a one-base difference, and the method using restriction enzymes requires polymorphism of the site for recognition by enzymes. Thus, the assay systems available today are not suitable for use in routine work because of the many problems they have.

## DISCLOSURE OF INVENTION:

Under these circumstances, the present inventors conducted intensive studies in order to develop a new method of identifying HLA antigen types that is sensitive enough to be capable of detecting a one-base difference. As a result, the present inventors found that by using an acridinium ester (AE) labelled probe that hybridised with a PCR product made by the PCR method from mRNA or genomic DNA extracted from a sample of interest, precision and convenience could be remarkably enhanced and the assay time shortened substantially compared to the previous methods. The present invention has been accomplished on the basis of this finding.

One method of the present invention is intended to identify HLA types using a probe that is labelled with an acridinium ester of the following general formula (II) via a linker of the following general formula (I). More specifically, the method comprises the following steps:

1. hybridizing labelled probes with the product of amplification of cDNA or genomic DNA prepared from mRNA in a sample of interest, each of said probes having a structure in which an acridinium ester of the general formula (II):

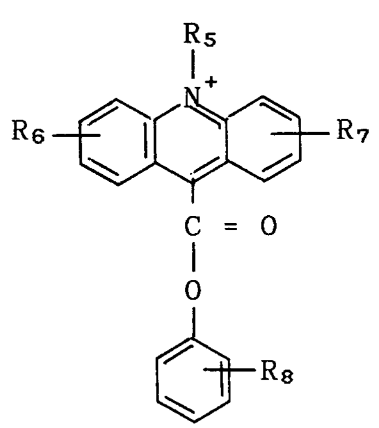

(II)

(where $R_5$ is an optionally substituted lower alkyl, lower alkenyl or allyl group; $R_6$ and $R_7$ which may be the same or different each represents a hydrogen atom, an amino group, a hydroxyl group, a thiol group, a halogen atom, a nitro group, an optionally substituted acetyl group, an optionally substituted lower alkyl

group, an optionally substituted lower alkenyl group, an optionally substituted allyl group, a lower alkoxy group or an allyloxy group; $R_8$ represents

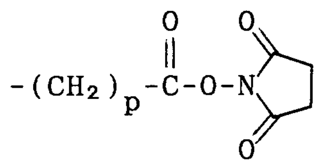

or

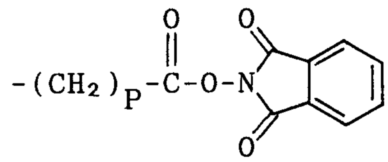

;

and p is an integer of 0 - 10) is labelled via a linker arm of the general formula (I):

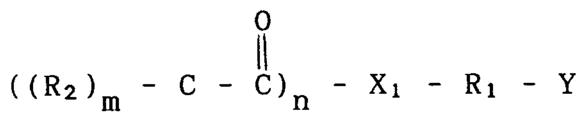

(I)

(where $X_1$ is a sulfur atom or an amino group; Y represents

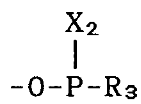

or

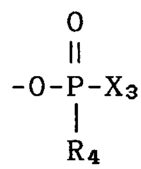

;

$R_1$ is an aliphatic hydrocarbon chain having 1 - 10 carbon atoms;

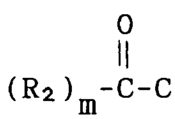

represents an $X_1$ protecting group, provided that m represents an integer of 1 - 3 that can be assumed by $R_2$, and $R_2$ represents one or more carbon atoms, oxygen atoms, nitrogen atoms or halogen atoms or a combination thereof which are such that $(R_2)_m$-C- will have an electron withdrawing group capable of protecting $X_1$; n is 1 or 2, provided that when $X_1$ is a sulfur atom, n is 1, and when $X_1$ is an amino group, n is 1 or 2; $X_2$ is a halogen atom or a substituted amino group; $X_3$ is a halogen atom, an amino group or -O-; $R_3$ is an optionally substituted lower alkyl, lower alkoxy or allyloxy group; and $R_4$ represents a lower alkyl or lower alkoxy, provided that when $X_3$ is -O-, $R_4$ represents a hydrogen atom);

2. selectively hydrolyzing the acridinium ester of nonhybridizing probes and those hybridizing probes which have a mismatch of at least one base in hybridization; and

3. measuring the amount of residual chemiluminescence.

Another method of the present invention for identifying HLA types comprises the following steps:

(1) hybridizing labelled probes to the product of amplification of cDNA prepared from mRNA in a sample of interest, said probes being constructed by labelling an acridinium ester of the general formula (II) on probes having a linker arm of the general formula (I) coupled thereto;

(2) selectively hydrolyzing the acridinium ester of nonhybridizing probes and those hybridizing probes which have a mismatch of at least one base in hybridization; and

(3) measuring the amount of residual chemiluminescence.

Still another method of the present invention comprises the following steps:

(a) extracting mRNA from a sample of interest;

(b) preparing cDNA by reverse transcription of the extracted mRNA;

(c) amplifying the constructed cDNA;

(d) hybridizing under hybridizing conditions the amplified cDNA to probes labelled with an acridinium

ester;

(e) selectively hydrolyzing the acridinium ester of nonhybridizing probes and those hybridizing probes that have a mismatch of at least one base in hybridization; and

(f) measuring the amount of residual chemiluminescence to identify the type of HLA in the sample of interest.

BRIEF DESCRIPTION OF THE DRAWINGS:

Fig. 1 shows the base sequences of DRB1 genes for amino acid numbers 5 - 31;

Fig. 2 shows the base sequences of DRB1 genes for amino acid numbers 32 - 62;

Fig. 3 shows the base sequences of DRB1 genes for amino acid numbers 63 - 94;

Fig. 4 is an HPLC chromogram of the reaction solution obtained when probes composed of oligonucleotides having an amino linker inserted thereinto were reacted with an acridinium ester, as well as a graph showing the amount of chemiluminescence from that solution;

Fig. 5 is a graph showing the difference between match and mismatch for bases in HLA DR4 as observed in the HPA method of the present invention that targeted a synthetic oligonucleotide using acridinium ester labelled probes specific to HLA Dw4;

Fig. 6 is a graph showing the difference between match and mismatch for bases in HLA DR4 as observed in the HPA method of the present invention that targeted a synthetic oligonucleotide using acridinium ester labelled probes specific to HLA Dw10;

Fig. 7 is a graph showing the difference between match and mismatch for bases in HLA DR4 as observed in the HPA method of the present invention that targeted a synthetic oligonucleotide using acridinium ester labelled probes specific to HLA Dw14;

Fig. 8 is a set of graphs showing the profile of residual chemiluminescence from the respective targets used in the HPA method of the present invention that was performed on the PCR amplified nucleotide sequences containing the hypervariable region of DR in genomic DNA; and

Fig. 9 is a set of graphs showing the profile of residual chemiluminescence from the respective targets used in the HPA method of the present invention that was performed on mRNA that had been extracted from cells, subjected to reverse transcription and amplified by the PCR method.

BEST MODE FOR CARRYING OUT THE INVENTION:

The methods of the present invention for identifying HLA types are described below in detail.

The HLA species that are targets of type identification by the methods of the present invention may be HLA-A, B and C of class I or HLA-DR, DQ and DP of class II. The methods are applicable to any of the specificities that were approved at the 10th International Histocompatibility Workshop in 1987. Among those target antigens, HLA-DR genes (DRB1) contain pseudo genes and present considerable difficulty in type identification. The specificities of HLA-DR antigens and the base sequences of their gene are shown in Figs. 1 - 3.

Extraction of mRNA may be performed by common methods such as: (1) lysing cells with a surfactant such as NP-40 and extracting mRNA with phenol or some other suitable agent [S.L. Berger, Method in Enzymology, 152, 227 - 234 (1987)]; (2) using guanidine thiocyanate [P. Chomcynski and N. Sacchi, Analytica Biochemistry, 162, 156 - 159 (1987)]; and (3) ultracentrifugation (F.M. Ausbel et al., Current Protocols in Molecular Biology 1).

Reverse transcription of the extracted mRNA to prepare cDNA may be performed using suitable reverse transcriptases such as AMV reverse transcriptase and M-MLV reverse transcriptase.

Amplification of cDNA may be performed by various methods, such as the PCR method in which using an oligo-dT primer or a random primer, mRNA is converted to a single-stranded DNA (sscDNA) with a reverse transcriptase and the sscDNA is amplified in a specific way, and a method using T7 polymerase and a reverse transcriptase. In the second method using T7 polymerase and a reverse transcriptase, the double-stranded DNA is denatured to a single-stranded DNA, which is then linked to a primer (i.e., an oligonucleotide having, with the DNA region to be amplified being interposed, a sequence complementary to the (+) and (-) strands, as well as a sequence complementary to the T7 promoter region), and a buffer solution containing ribonucleotide triphosphate, deoxyribonucleotide triphosphate, reverse transcriptase, T7 polymerase and any salts that are necessary for the enzyme used. Subsequently, the mixture is incubated at 37°C for several minutes, denatured at an elevated temperature (ca. 95°C), cooled to room temperature, mixed with a buffer solution containing reverse transcriptase, T7 polymerase and RNase H, and further incubated at 37°C for several hours.

The primers to be used in the PCR method are those which are so designed as to enable amplification of the hypervariable region to be detected with probes. A preferred length of the primers is 20 - 25 mer.

Probes for assaying can be synthesized with a nucelic acid synthesizer (Model 380B of Applied Biosystems). The probes are designed in such a way that the hypervariable part is located at the center, into which a linker having an amino group is inserted. The synthesized oligonucleotide is purified in the usual manner and mixed with an acridinium ester (AE) having an active ester dissolved in an organic solvent in a reaction solution containing dimethyl sulfoxide and a buffer solution such as HEPES. As a result, the active ester reacts with the amino group in the linker to effect labelling of the oligonucleotide with AE.

In order to insure that a negative type is not identified as "false-positive" by the methods of the present invention, the probes to be used must be selected in such a way as to meet the following criteria: for a negative case, the amount of residual chemiluminescence that is emitted after hydrolysis for a predetermined time is attenuated to the level for water which is a negative control, and for a positive case, the amount of residual chemiluminescence is at least 5 times, preferably at least 10 times, as high as the level for the negative case. As a result of the intensive studies conducted in order to select probes having these properties, the present inventors found that probes whose length and Tm value [(the number of bases G and C in a probe) x 4 + (the number of bases A and T in the probe) x 2] were within specified ranges proved particularly effective in the identification of HLA types. Stated more specifically, probes with a length of 19 - 28 mer and a Tm value of 62 - 80, preferably in the neighborhood of 72, are used in the present invention with particular preference. Examples of suitable probes are listed in Table 1 together with their base sequences and Tm values.

Table 1

| Probe No. | Base sequence | Tm |
|---|---|---|
| P6 | GGC AGC TTA AG# TTT GAA TGT CAT TTC TT | 76 |
| P7 | GTG GCA GCC TAA GA# GGG AGT GTC ATT | 80 |
| P8 | CTT GGA GTA CT# CTA CGT CTG AGT G | 72 |
| P9 | GAA GCG GGG CC# GGG TGG ACA AC | 76 |
| P10 | GGA GCA GGT TAA AC# ATG AGT GTC ATT | 74 |
| P11 | CGG CCT GAT GA# GGA GTA CTG GA | 70 |
| P12 | GGA AGA CG# AGC GGG CCG CG | 68 |
| P13 | GGT GGA CAC CGT #GT GCA GAC AC | 72 |
| P14 | ACT ACG GGG C#T GTG GAG AG | 62 |
| P15 | CTA CGG GGT TGG #TG AGA GCT TCA C | 76 |
| P16 | GAG TAC TCT ACG GG# TGA GTG TTA T | 70 |
| P17 | CTG GAA AGA CGC #GT CCA TAA CCA | 70 |

Notes: 1. Tm: GC × 4 + AT × 2

2. # represents a linker arm.

These probes are enclosed with solid lines in Figs. 1 - 3. The probes enclosed with dashed lines in

those figures are also usable in the methods of the present invention.

In actual assay, the amplified target is heated or alkali-treated to form a single strand, which was mixed with the probes and hybridized in the usual manner. The hybridization is performed in an acidic buffer solution. To shorten the reaction time, the hybridization may be effected in an acidic buffer solution containing a lithium salt at a temperature of 50 - 70 ° C for a period of from one minute to less than an hour. After the hybridization, a weak alkali solution containing a surfactant is added. When the base, sequence of the target is in fully complementary with those of the probes, the acridinium ester is protected against hydrolysis, but in the presence of a certain mismatch, the ester is hydrolyzed to cause attenuation of chemiluminescence.

The linker arm represented by the general formula (I) which is used in the present invention is already known by being disclosed in, for example, European Patent Publication EP 0310312, and the AE represented by the general formula (II) is also known by being disclosed in, for example, International Patent Publication WO89/02476.

The following examples are provided for the purpose of further illustrating the present invention but are in no way to be taken as limiting. In the following examples, identification of HLA types was performed on DR genes that were so selected as to differ from one another by only one base in order to demonstrate the significant advantages of the methods of the present invention. The AE used in the examples was a compound represented by the following structural formula:

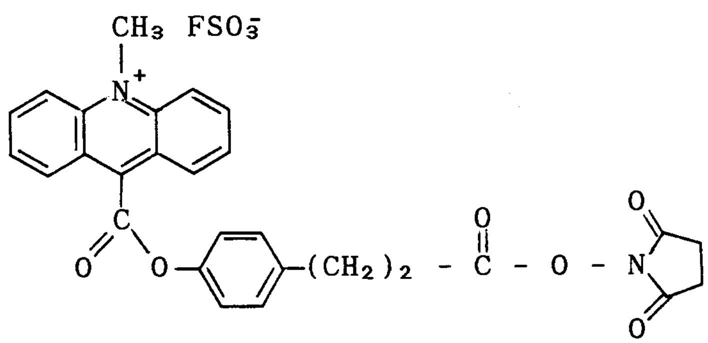

Chemical name: 4-(2-Succinimidyloxycarbonylethyl)phenyl-10-methylacridinium-9-carboxylate fluorosulfonate

Example 1

(a) Preparing Probes

As shown in Table 2, a DR antigen serologically identified as DR4 is further split into Dw4, Dw10, Dw13, Dw14, Dw15 and DKT2 at the molecular level. Among these subtypes, Dw4, Dw10 and Dw15 were used in the present assay. The base sequence of Dw4 differs from that of Dw14 by only one base and if such a method that is capable of detecting this difference in a convenient way, it is applicable to the identification of all antigen types.

Table 2

| Differences in Nucleotide and Amino Acid Sequences between DR4 Subtypes | | | | | | | | |
|---|---|---|---|---|---|---|---|---|
| Amino acid No. | 37... | 57... | 67 | 69 | 70 | 71... | 74... | 86 |
| Dw4 | Tyr<br>TAC | Asp<br>GAT | Leu<br>CTC | Glu<br>GAG | Gln<br>CAG | Lys<br>AAG | Ala<br>GCG | Gly<br>GGT |
| Dw10 | - | - | Ile<br>A | -<br>A | Asp<br>GC | Glu<br>G | - | Val<br>TG |
| Dw14 | - | - | - | - | - | Arg<br>G | - | Val<br>TG |
| Dw15 | - | Ser<br>AGC | - | - | - | Arg<br>G | - | - |
| KT2 | Ser<br>C | - | - | - | - | Arg<br>G | Glu<br>A | Val<br>TG |
| TAS and Dw13 | - | - | - | - | - | Arg<br>G | Glu<br>A | Val<br>TG |

Probes were synthesized with a nucleic acid synthesizer (Model 380B of Applied Biosystems) that was so designed as to insert an amino linker (Amino Modifier II of CLONTECH Laboratories, Inc.) of the general formula (I) in the neighborhood of a difference in base sequence as illustrated below:

P1 $^{3}$ GAC CTC GTC T*TC GCC CGG CG $^{5}$

P2 $^{3}$ GAC CTT CTG *CTC GCC CGG CG $^{5}$

P3 $^{3}$ GAC CTC GTC TC*C GCC CGG CG $^{5}$

*: amino linker

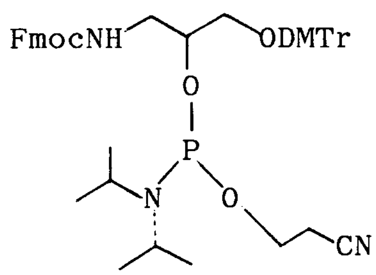

After the synthesis, 15% polyacrylamide electrophoresis was conducted and bands of interest were cut out and subjected to extraction with 0.5 M ammonium acetate and 1 mM EDTA overnight. The extracts were purified on Sep-pack C18 column. The purified oligonucleotides were distributed among Eppendorf tubes in respective amounts of ca. 0.5 OD. After vacuum drying, 4 µℓ of water, 1 µℓ of 1 M HEPES (pH 8), 3 µℓ of DMSO and 2 µℓ (0.8 mg/50 µℓ DMSO) of AE were added and reaction was performed at 37°C for 20 minutes under lightshielding conditions. Again, 1.5 µℓ of water, 0.5 µℓ of 1 M HEPES and 3 µℓ of AE were added and the reaction was continued at 37°C for 20 minutes under light-shielding conditions. Then, in order to block the unreacted AE, a liquid mixture containing 125 µM lysine, 0.1 M HEPES and 50% DMSO was added and left to stand at room temperature for 5 minutes. By precipitation with ethanol, the free AE was removed.

Water (100 µℓ) was added to the precipitating probes and the whole amount was injected into TSK gel oligo DNA RP column (product of Tosoh Corp.; 4.6 mm i.d. x 15 cm L). The resulting HPLC chromatogram is shown in Fig. 4. The effluent was sampled at 0.5-minute intervals and the amount of chemiluminescence (in RLUs) from each fraction was measured to determine whether the oligonucleotides were correctly

labelled with AE.

The conditions of HPLC were as follows.

| | |
|---|---|
| Eluent A: | 50 mM TEAA (triethyl acetate ammonium) |
| B: | $CH_3CN$ (density gradient: 0 - 40% over 40 minutes) |
| Flow rate: | 1 mℓ/min. |
| Detector: | UV 260 nm. |

(b) Preparing Primers

In order to amplify the hypervariable region to be detected with the probes, primers were prepared with a nucleic acid synthesizer (Model 380B of Applied Biosystems) and purified in the same way as in the preparation of the probes. The primers had the following sequences.

Primer 4: 5'-TAC TGC AGA CAC AAC TAC GGG GTT-3'

Primer 5: 5'-GGA GTA CCG GGC GGT GAG GGA GC-3'

(2) Preparing and Extracting Targets

(a) Preparing synthetic oligonucleotides

Synthetic oligonucleotides to be used as targets were determined for base sequences in such a way that they would be complementary to P1, P2 and P3 and, based on the thus determined base sequences, the oligonucleotides were synthesized with a nucleic acid synthesizer. The synthesized oligonucleotides were purified by the same method as in the preparation of the probes.

(b) Extracting RNA from samples

(i) Extracting RNA from HTC (homozygous typing cells: cultured cells having a homozygous DR gene)

The cells used were BM14 having a homozygous Dw4 gene, FS having homozygous Dw10 gene, and THO having homozygous Dw14 gene. HTC were cultured in a 10% bovine serum supplemented RPMI 1640 medium. Cells ($5 \times 10^6$ - $10^7$) were washed with PBS three times. The HTC were suspended in 4.5 mℓ of a hypotonic buffer (10 mM Tris-HCℓ, pH 7.5, 10 mM NaCℓ, and 1.5 mM $MgCℓ_2$) was suspended in HTC and 250 μℓ of a vanadyl complex (200 mM) was added. Following the addition of 10% NP-40 (150 μℓ), the mixture was stirred and thereafter left to stand at 4°C for 5 minutes. Then, 100 μℓ of 10% SDS and 40 μℓ of 0.5 M EDTA was added. Following the addition of phenol (ca. 5 mℓ), the mixture was stirred for 5 minutes and centrifuged (5 minutes at 3,000 rpm). Extraction with phenol was repeated three more times. The supernatant was transferred into another tube and, after adding 100 μℓ of 5 M NaCℓ and cold ethanol, the supernatant was left to stand at -90°C overnight or in a Dry Ice box for 30 minutes. After centrifugation (20 minutes at 3,000 rpm), the supernatant was discarded and the pellets were rinsed with ethanol and dried under vacuum. The dried pellets were dissolved in 600 μℓ of water and after adding an equal volume of 4 M LiCℓ, the solution was left to stand at 4°C overnight. After centrifugation (20 minutes x 15,000 rpm), the pellets were rinsed once with 4 M LiCℓ and once with ethanol, and dried. The dried pellets were dissolved in 100 μℓ of water and the amount of RNA was calculated from absorbance data.

(ii) Extracting RNA from lymphocytes in fresh blood

To ca. 4 mℓ of heparinized blood, an equal volume of isotonic buffer solution was added and the mixture was stirred well in a pipette. 6-mℓ of Ficoll-Paque (a specific gravity solution of Pharmacia) was dispensed into a 15-mℓ centrifugal tube and the blood sample was plated gently. Centrifugation was performed at room temperature (40 - 50 minutes at 400 g). Lymphocytes in the intermediate layer were collected carefully and ca. 6 mℓ of an isotonic buffer solution was added. Following gentle stirring, the mixture was centrifuged at 60 - 100 g for 10 minutes. The supernatant was discarded and the pellets were treated as in (i) to extract RNA.

(3) Reverse Transcription of mRNA (preparing sscDNA) and its Specific Amplification

To 1 μℓ of RNA, 0.5 μℓ of oligo (dT) was added and diluted with water to make 20 μℓ. After standing at 65°C for 5 minutes, the solution was cooled to room temperature and mixed with 10 mM dNTP mix and

a reverse transcriptase buffer having five times as high concentration. Further, 1 μℓ of an M-MLV reverse transcriptase (200 units; Bezesda Research) was added and reaction was performed at 37°C for 60 minutes. After standing at 65°C for 10 minutes, 180 μℓ of TE buffer was added and the mixture was stored at -90°C.

To 10 μℓ of sscDNA, 5 μℓ of PCR buffer having ten times as high concentration, 1 μℓ of 10 mM dNTP, 5 μℓ of each primer (10 pmol/μℓ), 24 μℓ of water and 0.5 μℓ of a Taq polymerase ("Ampli Taq" of Takara Shuzo) were added. After denaturation at 92°C for 5 minutes, a heat treatment was conducted through 45 cycles of heating at 65°C for 3 minutes and at 92°C for 2 minutes. In the last stage, the mixture was left to stand at 65°C for 7 minutes. A 2-μℓ aliquot of the mixture was subjected to HPA (homogeneous protection assay).

(4) Hybridization of Probes and Targets

Eppendorf tubes were dispensed with 15 μℓ of 2 x hybrid buffer (see Note 1), 5 μℓ of 2 x transport media (see Note 2), 3 μℓ of probe ($10^6$ - $10^7$ RLUs) and targets and the mixture was incubated at 60°C for 60 minutes. After hybridization, 270 μℓ of Mock Hybrid Mix (see Note 3) was added to prepare hybrid solutions. Water was used as a negative control.

Notes:

1. 2 x Hybrid Buffer: 0.2 M lithium succinate pH 5.2, 18% lithium lauryl sulfate, 2 mM EDTA/EGTA
2. 2 x Transport Media: 6% lithium lauryl sulfate, 60 mM sodium phosphate buffer, pH 6.8 2 mM EDTA/EGTA
3. Mock Hybrid Mix (in 20 mℓ)

| | |
|---|---|
| 2 x Hybrid Buffer | 10.0 mℓ |
| 2 x Transport Media | 3.33 mℓ |
| 0.01 M NaOAc (pH 5), 0.1% SDS | 2.0 mℓ |
| $H_2O$ | 4.67 mℓ |

(5) HPA Experiment

By treatment with solution A composed of 0.2 M sodium tetraborate (pH 7.6) and 5% Triton X-100 (surfactant of Wako Pure Chemical Industries, Ltd.), chemical sections were added to the hybrid solutions. A comparison of RLU attenuation was performed to check for matches and mismatches in base sequences. The term "mismatch" means the presence of a difference in one or more bases in hybridization. To tubes preloaded with 100 μℓ of solution A, 15 μℓ of hybrid solutions were added and the mixtures were incubated at 60°C for 10 minutes, cooled for 30 seconds and left to stand at room temperature for 1 minute, followed by measurement of the amount of chemiluminescence. The amount of residual chemiluminescence was calculated in relative terms, with 100% being the 0-minute value, or the amount of chemiluminescence that was measured immediately after adding 15 μℓ of hybrid solutions to 100 μℓ of cooled solution A.

Results and Discussion

(1) HPA on synthetic oligonucleotides as targets

The results of HPA on synthetic oligonucleotides that were used as targets are shown in Figs. 5, 6 and 7.

Fig. 5 is a graph showing the differences in match and mismatch of bases in HLA DR4 when tested by the HPA method using AE labelled probes (P1) specific to HLA Dw4. T1 (Dw4) matched completely with the base sequence of P1. T2 (Dw10) had 4-base mismatches whereas T3 (Dw14) had a 1-base mismatch. According to Fig. 5, when hybridization with P1 was performed for 1 hour, the residual value of RLUs at 10 minutes selection was 75% in T1 that completely matched with the probes, 41% in T3 having a 1-base mismatch, and 10% in T2 having 4-base mismatches. This shows the ability of the methods of the present invention to correctly detect even a one-base difference in HLA.

Substantially the same results were attained when P1 was replaced by P2 and P3 that were AE labelled probes specific to HLA Dw10 and Dw14, respectively.

Fig. 6 is a graph showing the differences in match and mismatch of bases in HLA DR4 when tested by

the HPA method using AE labelled probes (P2) specific to HLA Dw10. T2 (Dw10) matched completely with the base sequence of P2. T1 (Dw4) had 4-base mismatches whereas T3 (Dw14) had 5-base mismatches. According to Fig. 6, when hybridization with P2 was performed for 1 hour, the residual value of RLUs at 10 minutes selection was 72% in T2 that completely matched with the probes, 0.5% in T1 having 4-base mismatches, and 2% in T3 having 5-base mismatches. It was therefore clear that the methods of the present invention were also capable of correctly detecting the differences in DR bases in HLA of class II even when probes P2 were used.

Fig. 7 is a graph showing the differences in match and mismatch of bases in HLA DR4 when tested by the HPA method using AE labelled probes (P3) specific to HLA Dw14. T3 (Dw14) matched completely with the base sequence of P3. T1 (Dw4) had a 1-base mismatch whereas T2 (Dw10) had 5-base mismatches. According to Fig. 7, when hybridization with P3 was performed for 1 hour, the residual value of RLUs at 10 minutes selection was 66% in T3 which matched completely with the probes, 38% in T1 having a 1-base mismatch, and 12% in T2 having 5-base mismatches. It was therefore clear that the methods of the present invention were also capable of correctly detecting the differences in DR bases in HLA of class II even when probes P3 were used.

Thus, it was verified that the methods of the present invention are capable of correctly detecting differences as small as one base in targets that are synthetic oligonucleotides having the same base sequence as DR4.

(2) HPA on genomic DNA as targets

The base sequence of genomic DNA containing the DR hypervariable region was amplified by the PCR method and subjected to HPA. The results are shown in Fig. 8. The conditions of HPA were as follows: each probe was added in an amount of ca. $10^7$ RLUs, hybridization was performed for 1 hour, and selection was performed at 10 minutes. Symbols T1, T2 and T3 in Fig. 8 represent synthetic nucleotides as in Figs. 5 - 7, and BM14, FS and THO represent cell names. As is clear from Fig. 8, the type of Dw10 could be correctly identified using probes P2 but when P1 was used, FS having 4-base mismatches exhibited a high level of RLUs whereas THO having a complementary sequence could not be identified using P3. Thus, correct type identification of system using genomic DNA was impossible depending on the kind of target and this would be due to the influence of pseudo genes.

(3) HPA on mRNA as targets

The results of HPA conducted on mRNA that was extracted from cells and subjected to reverse trnascription and amplification by PCR are shown in Fig. 9. Symbols T1, T2, T3, BM14, FS and THO in Fig. 9 have the same meanings as in Fig. 8. As is clear from Fig. 9, the types of both Dw10 and Dw14 were successfully identified although in systems using genomic DNA, the type of Dw10 could not be correctly identified and it was impossible to identify the type of Dw14. Presumably, the adverse effects of pseudo genes can be eliminated when mRNA is used as a sample and this conclusion is also suggested from the complete parallelism between the data of residual RLUs and the results with the pure system. Thus, it was verified that DR subtypes can be correctly identified by using as targets the amplified products of cDNA that was prepared by reverse transcription of mRNA.

Example 2 : HLA DR Type Identification Using Lymphocytes in Peripheral Blood

(1) Probes

Twelve probes, P6 - P17, that are enclosed with solid lines in Figs. 1 - 3 and which are also shown in Table 1 were used in evaluating the effectiveness of the methods of the present invention.

(2) Extracting RNA from lymphocytes in peripheral blood

Fresh blood was sampled in a 5-mℓ portion with a heparinized syringe and lymphocytes were separated with Ficoll-Paque and washed with PBS twice. To the washed lymphocytes, 1 mℓ of solution D (see Note 1) was added and the mixture was stirred with a vortex mixer (IKEDA SCIENTIFIC Co., Ltd.) for 2 minutes. To the stirred mixture, 0.1 mℓ of 2 M $CH_3COONa$ (pH 4), 1 mℓ of water-saturated phenol and 0.2 mℓ of $CHC\ell_3$ were added and stirred with a vortex mixer for 30 seconds. After centrifugation at 3,000 rpm (for 10 minutes at 4 °C), the supernatant was transferred into Eppendorf tubes. After adding an equal

volume of isopropanol, the mixture was left to stand in a deep freezer (ULTRA LOW of Sanyo, -135 °C) for 15 minutes. After centrifugation at 12,000 g (for 15 minutes at 4 °C), 70% ethanol was added to the precipitate, which was further centrifuged at 12,000 g (for 5 minutes at 4 °C). After addition of 100% ethanol, the precipitate was further centrifuged for 5 minutes and dried. The dried product was dissolved in 100 μℓ of $H_2O$ to prepare an RNA solution.

(3) Reverse transcription and amplification of RNA

A mixture for reverse transcription (see Note 2) and $H_2O$ were added to 10 μℓ of the RNA solution to make a total volume of 90 μℓ. After standing at 65 °C for 5 minutes, the mixture was cooled to room temperature and mixed with 1 μℓ of M-MLV transcription (BRL; 200 units/μℓ), followed by incubation at 37 °C for 60 minutes. After standing at 65 °C for 10 minutes, 10 μℓ of PCR mixture (see Note 3) was added. After denaturation at 92 °C for 2 minutes, amplification was conducted through 45 cycles of heating at 92 °C for 2 minutes, 55 °C for 1.5 minutes and 72 °C for 2 minutes. Thereafter, extension was conducted for 8 minutes.

(4) Assay by the HPA method

A probe solution (see Note 4) was distributed in 50-μℓ portions (ca. 1,000,000 RLUs) in Falcon tubes (see Catalog No. 2053) with care being taken to avoid contact with the side wall. The amplified samples were heated at 95 °C for 3 minutes, replaced on ice and added in 5-μℓ portions under stirring. After incubation that lasted for exactly 60 minutes at 60 °C, a selection reagent (see Note 5) was distributed in 200-μℓ portions, followed by incubation for an additional 15 minutes.
Notes:

1. Solution D: 4 M guanidine thiocyanate, 25 mM sodium citrate pH 7, 0.5% salukosyl, 0.1 M 2 ME
2. Mixture for reverse transcription:
1 μℓ random primer (0.5 μg/μℓ), Takara Shuzo
2 μℓ 10 mM dNTP mixture, Takara Shuzo
10 μℓ 10 mM PCR Buffer (100 mM Tris-HCℓ pH 8.3,
500 mM KCℓ, 25 mM $MgCℓ_2$)
3. PCR mixture:
2.5 μℓ primer 14 (20 pmol/μℓ)
[Primer 14: 5'-TGG GGA CAC CCG ACC ACG TTT C-3']
2.5 μℓ primer 15 (20 pmol/μℓ)
[Primer 15: 5'-CTC GCC GCT GCA CTG TGA AGC T-3']
4.5 μℓ $dH_2O$, 0.5 μℓ ampli-Taq of Takara Shuzo
4. Probe solution: Probes diluted with a 1:1 mixture of a hybridization solution and transport media
hybridization solution: 0.2 M lithium succinate pH 5.2, 18% lithium lauryl sulfate, 2 mM EDTA/EGTA
transport media: 6% lithium lauryl sulfate, 60 mM sodium phosphate buffer pH 6.8, 2 mM EDTA/EGTA
5. Selection reagent: 0.2 M sodium tetraborate pH 7.6, 5% Triton X-100

Results and Discussion

The results are shown in Table 3. With the value for $H_2O$ being taken as a negative control, samples that exhibited chemiluminescence (RLUs) in amounts at least 5 times as high as the negative control were rated as "positive". With reference to the base sequences shown in Figs. 1 - 3, subtypes were estimated as shown in Table 4, which also shows the results of type identification by a conventional serological method. As Table 4 shows, the results of HPA method were in complete agreement with those obtained by the serological method. Thus, it was verified that successful type identification can be achieved by the HPA method using peripheral lymphocytes having a heterozygous gene. Closer type identification is impossible by the serological procedure but possible by the HPA method if more probes are used.

Hence, the HPA method of the present invention enables various antigen types and subtypes to be identified in a more convenient and precise manner than the prior art methods.

Table 3

| Probe / Sample | P6 | P7 | P8 | P9 | P10 | P11 | P12 | P13 | P14 | P15 | P16 | P17 |
|---|---|---|---|---|---|---|---|---|---|---|---|---|
| $H_2O$ | 2166 | 1215 | 1278 | 1387 | 1440 | 1245 | 1287 | 2545 | 2815 | 1401 | 1128 | 8142 |
| 1 | 1820 | 26969 | 1093 | 2312 | 29071 | 1319 | 1254 | 4181 | 4838 | 91210 | 1061 | 3486 |
| 2 | 85942 | 61582 | 1100 | 3560 | 2148 | 1507 | 1118 | 2671 | 3579 | 163007 | 1099 | 4872 |
| 3 | 3114 | 1047 | 8045 | 2053 | 44136 | 1284 | 2454 | 4792 | 4335 | 30608 | 1107 | 6599 |
| 4 | 2224 | 1032 | 941 | 1346 | 1459 | 1167 | 1666 | 2356 | 3759 | 151732 | 17552 | 3578 |
| 5 | 2759 | 1061 | 899 | 2016 | 1196 | 1093 | 1167 | 2974 | 106578 | 62832 | 30603 | 2204 |
| 6 | 88533 | 1281 | 25699 | 2893 | 3619 | 1154 | 1581 | 5901 | 4053 | 83237 | 1050 | 2583 |
| 7 | 2652 | 983 | 11861 | 2242 | 33747 | 1085 | 60396 | 2910 | 3730 | 82149 | 1001 | 2292 |
| 8 | 1926 | 1027 | 881 | 1400 | 40707 | 1053 | 1159 | 2630 | 3816 | 104028 | 13489 | 2071 |
| 9 | 2040 | 40291 | 890 | 3572 | 1888 | 1211 | 1232 | 2744 | 83450 | 85817 | 23887 | 3543 |
| 10 | 1861 | 1902 | 13476 | 2801 | 33268 | 48561 | 1695 | 3634 | 3248 | 141692 | 1095 | 4875 |

Table 4

| | Serological method | | HPA method | |
|---|---|---|---|---|
| 1 | DR2 | DR4 | DR2 | DR4 |
| 2 | DR1 | DR2 | DR1 (Dw1) | DR2 |
| 3 | DR4 | DRw14 | DR4 | DRw14 |
| 4 | DR8 | DR8 | DR8 | DR8 |
| 5 | DR8 | DRw12 | DR8 | DRw12a or b |
| 6 | DR1 | DRw14 | DR1 | DRw14 |
| 7 | DR4 | DRw13 | DR4 | DRw13 |
| 8 | DR4 | DR8 | DR4 | DR8 |
| 9 | DR2 | DRw12 | DR2 | DRw12a or b |
| 10 | DR4 | DR5 | DR4 | DR5 (DRw11a or b) |

Example 3 : Type Identification Using Genomic DNA

In order to perform precise identification of HLA DR types, probes capable of detecting the second and third hypervariable regions of DRB1 gene are necessary. Since these regions contain many base sequences that are identical to those of similar genes, it is necessary to use mRNA as discussed below. On the other hand, the base sequences of the first hypervariable region of HLA DRB1 and the hypervariable regions of DQ and DP differ so greatly from those of similar genes that genomic DNA can be used when those regions are to be assayed.

(1) Preparing probes

Probes used in identifying the types of HLA DRB1 were P10 that was complementary to DR4 and P11 that was complementary to DR11. For identifying the types of DQB, PQ1 that was complementary to DQw7 and PQ2 that was complementary to DQw3 (DQw7, 8 and 9) were prepared in the same way as in Example 1.

PQ1: 5'-AGC GCG TGC GTT *AT GTG ACC AG-3'

PQ2: 5'-AGA GGA GTA CGC *AC GCT TCG ACA G-3'

*: Amino linker

(2) Extracting and amplifying genomic DNA

Two types of HTC, i.e., THO having homozygous DR4 and DQw3 and BM21 having homozygous DR11 and DQw7, were used in the experiment. These HTCs were cultured in an RPMI-1640 medium containing 10% FCS and 5 X $10^6$ - $10^7$ cells were washed with PBS twice. Thereafter, genomic DNA was extracted with Nucleic Acid Extractor Model 340A (Applied Biosystems).

To 1 $\mu\ell$ of the extracted genomic DNA, 50 pmol of an amplifying primer (see below), 20 mM of dNTP, 10 $\mu\ell$ of 10 x PCR buffer, and 0.5 $\mu\ell$ of Ampli-Taq were added to make a total volume of 100 $\mu\ell$. After denaturation at 92°C for 2 minutes, amplification was performed through 35 cycles of heating at 92°C for 2 minutes, 55°C for 1.5 minutes and 72°C for 2 minutes. Thereafter, extension was performed at 72°C for 8 minutes.

Primer set for DRB1 amplification:

5'-CCC CAC AGC ACG TTT CTT G-3'

5'-CCG CTG CAC TGT GAA GCT CT-3'

Primer set for DQB1 amplification:

5'-GCA TGT GCT ACT TCA CCA ACG-3'

5'-CTG GTA GTT GTG TCT GCA CAC-3'

(3) Assay by the HPA method

The procedures of Example 2 were followed.

Results and Discussion

The results of measurements are shown in Table 5.

Table 5

| | DR | | DQ | |
|---|---|---|---|---|
| | P10 | P11 | PQ1 | PQ2 |
| $H_2O$ | 2,578 | 488 | 1,708 | 4,480 |
| THO | 62,418 | 461 | 709 | 77,049 |
| BM21 | 889 | 148,518 | 100,790 | 115,658 |
| Note: The numerals represent the amount of residual chemiluminescence in RLUs. | | | | |

When P10 was used, THO having a complementary sequence exhibited an RLUs value significantly higher than the negative control ($H_2O$) whereas BM21 which did not have a complementary sequence exhibited a lower value than the negative control. In contrast, when P11 was used, BM21 having a complementary sequence exhibited a high RLUs value whereas THO which did not have a complementary sequence exhibited a comparable value to the negative control.

When PQ1 was used, BM21 having a complementary sequence exhibited a high RLUs value but THO which did not have a complementary sequence exhibited a lower value than the negative control. When PQ2 was used, both THO and BM21 having a complementary sequence exhibited higher values than the negative control.

Thus, some of DRB1 types, as well as DQ and DP types can be identified by the methods of the present invention using genomic DNA.

The methods of the present invention (HPA procedure) for identifying human leukocyte types are compared in Table 6 with the conventional methods (a serological method and a cytological method).

Table 6

| Comparison of Three HLA Assay Methods | | | |
|---|---|---|---|
| | HPA | Serological | Cytological |
| Blood sample | 1 - 5 mℓ | 30 mℓ | 30 mℓ |
| Assay time | 1 day | 1 day | 1 week |
| Precise classification | possible | impossible | possible |
| Procedure | simple | complicated | complicated |
| Accuracy | high | low | low |
| Isotope | not necessary | not necessary | necessary |
| Applicability in routine laboratories | yes | no | no |

The aforementioned methods of the present invention for HLA type identification not only enable the heretofore serologically unachievable identification of DR subtypes but also solve all the problems associated with the currently attempted methods of type identification with the aid of genes. According to the methods of the present invention, not only HTCs having homozygous genes but also peripheral lymphocytes having heterozygous genes can be used in type identification. Needless to say, the methods can be used not only to identify DR types but also genes of class II such as DQ and DP, and genes of class I such as A, B and C.

**Claims**

1. A method of identifying various types of human leukocyte antigens (HLA) which comprises the following steps:
   (1) hybridizing labelled probes with the product of amplification of cDNA prepared from mRNA in a sample of interest, each of said probes having a structure in which an acridinium ester of general of the formula (II):

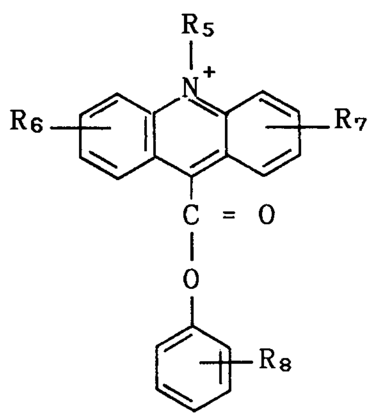

(II)

(where $R_5$ is an optionally substituted lower alkyl, lower alkenyl or allyl group; $R_6$ and $R_7$ which may be the same or different each represents a hydrogen atom, an amino group, a hydroxyl group, a thiol group, a halogen atom, a nitro group, an optionally substituted acetyl group, an optionally substituted lower alkyl group, an optionally substituted lower alkenyl group, an optionally substituted allyl group, a lower alkoxy group or an allyloxy group; $R_8$ represents

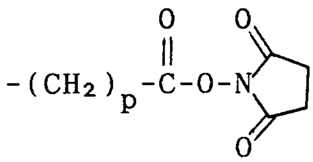 or 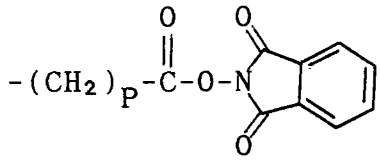 ;

and p is an integer of 0 - 10) is labelled via a linker arm of the general formula (I):

$$((R_2)_m - C - \overset{\overset{O}{\|}}{C})_n - X_1 - R_1 - Y \qquad (I)$$

(where $X_1$ is a sulfur atom or an amino group; Y represents

$$-O-\overset{\overset{X_2}{|}}{P}-R_3 \quad \text{or} \quad -O-\overset{\overset{O}{\|}}{\underset{\underset{R_4}{|}}{P}}-X_3 \ ;$$

$R_1$ is an aliphatic hydrocarbon chain having 1 - 10 carbon atoms;

$$(R_2)_m-\overset{\overset{O}{\|}}{C}-C$$

represents an $X_1$ protecting group, provided that m represents an integer of 1 - 3 that can be assumed by $R_2$, and $R_2$ represents one or more carbon atoms, oxygen atoms, nitrogen atoms or halogen atoms or a combination thereof which are such that $(R_2)_m$-C- will have an electron withdrawing group capable of protecting $X_1$; n is 1 or 2, provided that when $X_1$ is a sulfur atom, n is 1, and when $X_1$ is an amino group, n is 1 or 2; $X_2$ is a halogen atom or a substituted amino group; $X_3$ is a halogen atom, an amino group or -O-; $R_3$ is an optionally substituted lower alkyl, lower alkoxy or allyloxy group; and $R_4$ represents a lower alkyl or lower alkoxy, provided that when $X_3$ is -O-, $R_4$ represents a hydrogen atom);
(2) selectively hydrolyzing the acridinium ester of nonhybridizing probes and those hybridizing probes which have a mismatch of at least one base in hybridization; and
(3) measuring the amount of residual chemiluminescence.

**2.** A method of identifying various types of human leukocyte antigens (HLA) which comprises the following steps:
(1) hybridizing labelled probes with the product of amplification of cDNA prepared from mRNA in a sample of interest, each of said probes having a structure in which an acridinium ester of the general formula (II):

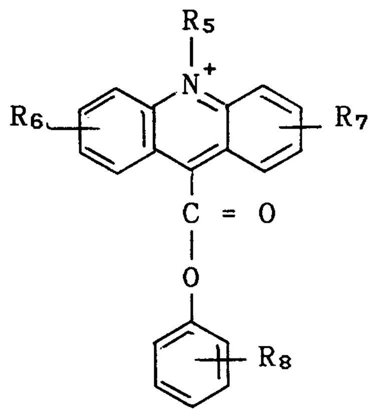

(II)

(where $R_5$ is an optionally substituted lower alkyl, lower alkenyl or allyl group; $R_6$ and $R_7$ which may be the same or different each represents a hydrogen atom, an amino group, a hydroxyl group, a thiol group, a halogen atom, a nitro group, an optionally substituted acetyl group, an optionally substituted lower alkyl group, an optionally substituted lower alkenyl group, an optionally substituted allyl group, a lower alkoxy group or an allyloxy group; $R_8$ represents

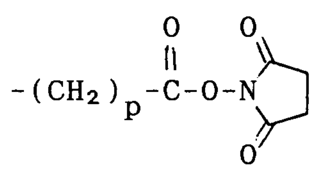 or 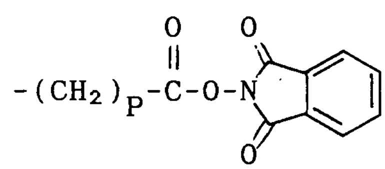 ;

and p is an integer of 0 - 10) is labelled via a linker arm of the general formula (I):

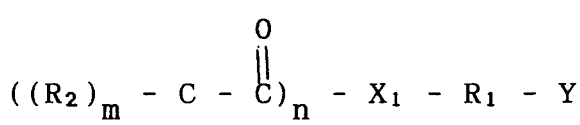

(I)

(where $X_1$ is a sulfur atom or an amino group; Y represents

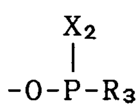 or 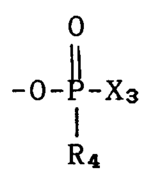 ;

$R_1$ is an aliphatic hydrocarbon chain having 1 - 10 carbon atoms;

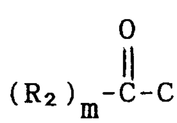

represents an $X_1$ protecting group, provided that m represents an integer of 1 - 3 that can be assumed by $R_2$, and $R_2$ represents one or more carbon atoms, oxygen atoms, nitrogen atoms or halogen atoms or a combination thereof which are such that $(R_2)_m$-C-will have an electron withdrawing group capable of protecting $X_1$; n is 1 or 2, provided that when $X_1$ is a sulfur atom, n is 1, and when $X_1$ is an amino group, n is 1 or 2; $X_2$ is a halogen atom or a substituted amino group; $X_3$ is a

halogen atom, an amino group or -O-; $R_3$ is an optionally substituted lower alkyl, lower alkoxy or allyloxy group; and $R_4$ represents a lower alkyl, lower alkoxy or allyloxy group, provided that when $X_3$ is -O-, $R_4$ represents a hydrogen atom); and
(2) selectively hydrolyzing the acridinium ester of nonhybridizing probes and those hybridizing probes which have a mismatch of at least one base in hybridization.

**3.** A method according to Claim 1 or 2 which is performed uniformly in solution.

**4.** A method according to any one of the preceding Claims in which HLA is an antigen of class II.

**5.** A method according to Claim 4 wherein the antigen of class II is a DR molecule.

**6.** A method of identifying various types of HLA which comprises the following steps:
(a) extracting mRNA from a sample of interest;
(b) preparing cDNA by reverse transcription of the extracted mRNA;
(c) amplifying the constructed cDNA;
(d) hybridizing under hybridizing conditions the amplified cDNA to probes labelled with an acridinium ester;
(e) selectively hydrolyzing the acridinium ester of nonhybridizing probes and those hybridizing probes that have a mismatch of at least one base in hybridization; and
(f) measuring the amount of residual chemiluminescence to identify the type of HLA in the sample of interest.

**7.** A method according to Claim 6 wherein step (d) is performed uniformly in solution.

**8.** A method according to Claim 6 or 7 wherein HLA is an antigen of class II.

**9.** A method according to Claim 8 wherein the antigen of class II is a DR molecule.

**10.** A method of identifying various types of human leukocyte antigens (HLA) which comprises the following steps:
(1) hybridizing labelled probes with the product of amplification of genomic DNA in a sample of interest, each of said probes being not complementary to pseudo genes and having a structure in which an acridinium ester of the general formula (II):

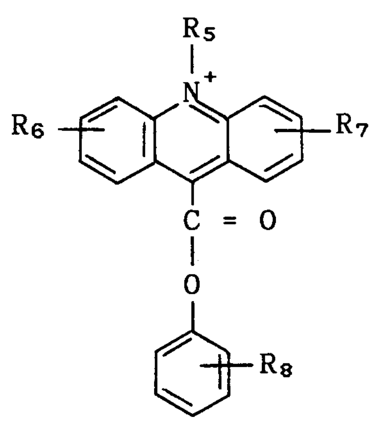

(II)

(where $R_5$ is an optionally substituted lower alkyl, lower alkenyl or allyl group; $R_6$ and $R_7$ which may be the same or different each represents a hydrogen atom, an amino group, a hydroxyl group, a thiol group, a halogen atom, a nitro group, an optionally substituted acetyl group, an optionally substituted lower alkyl group, an optionally substituted lower alkenyl group, an optionally substituted allyl group, a lower alkoxy group or an allyloxy group; $R_8$ represents

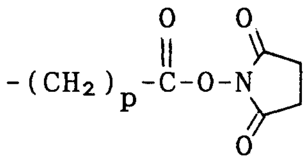 or 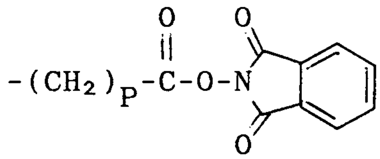 ;

and p is an integer of 0 - 10) is labelled via a linker arm of the general formula (I):

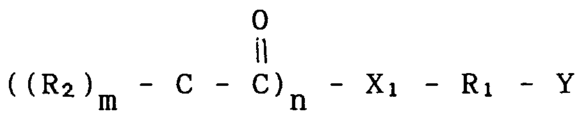 (I)

(where $X_1$ is a sulfur atom or an amino group; Y represents

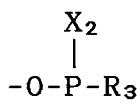 or 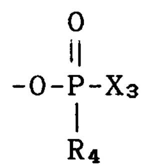 ;

$R_1$ is an aliphatic hydrocarbon chain having 1 - 10 carbon atoms;

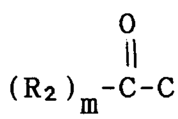

represents an $X_1$ protecting group, provided that m represents an integer of 1 - 3 that can be assumed by $R_2$, and $R_2$ represents one or more carbon atoms, oxygen atoms, nitrogen atoms or halogen atoms or a combination thereof which are such that $(R_2)_m$-C- will have an electron withdrawing group capable of protecting $X_1$; atom or a substituted amino group; $X_3$ is a halogen atom, an amino group or -O-; $R_3$ is an optionally substituted lower alkyl, lower alkoxy or allyloxy group; and $R_4$ represents a lower alkyl, lower alkoxy or allyloxy group, provided that when $X_3$ is -O-, $R_4$ represents a hydrogen atom);

(2) selectively hydrolyzing the acridinium ester of nonhybridizing probes and those hybridizing probes which have a mismatch of at least one base in hybridization; and

(3) measuring the amount of residual chemiluninescence.

**11.** A method of identifying various types of human leukocyte antigens (HLA) which comprises the following steps:

(1) hybridizing labelled probes with the product of amplification of cDNA prepared from mRNA in a sample of interest, each of said probes being not complementary to false genes and having a structure in which an acridinium ester of the general formula (II):

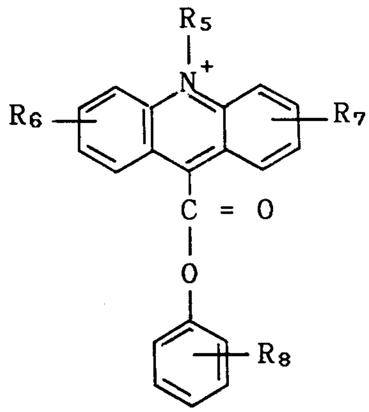

(II)

(where $R_5$ is an optionally substituted lower alkyl, lower alkenyl or allyl group; $R_6$ and $R_7$ which may be the same or different each represents a hydrogen atom, an amino group, a hydroxyl group, a thiol group, a halogen atom, a nitro group, an optionally substituted acetyl group, an optionally a hydroxyl group, a thiol group, a halogen atom, a nitro group, an optionally substituted acetyl group, an optionally substituted lower alkyl group, an optionally substituted lower alkenyl group, an optionally substituted allyl group, a lower alkoxy group or an allyloxy group; $R_8$ represents

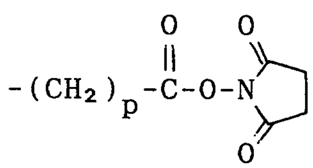 or 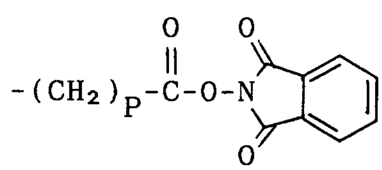 ;

and p is an integer of 0 - 10) is labelled via a linker arm of the general formula (I):

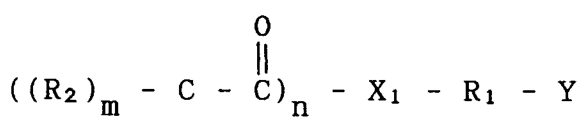

(I)

(where $X_1$ is a sulfur atom or an amino group; Y represents

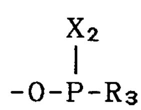 or 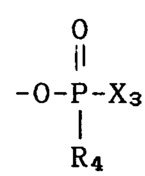 ;

$R_1$ is an aliphatic hydrocarbon chain having 1 - 10 carbon atoms;

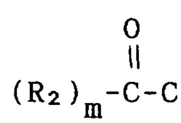

represents an $X_1$ protecting group, provided that m represents an integer of 1 - 3 that can be assumed by $R_2$, and $R_2$ represents one or more carbon atoms, oxygen atoms, nitrogen atoms or halogen atoms or a combination thereof which are such that $(R_2)_m$-C- will have an electron withdrawing group capable of protecting $X_1$; n is 1 or 2, provided that when $X_1$ is a sulfur atom, n is 1, and when $X_1$ is an amino group, n is 1 or 2; $X_2$ is a halogen atom or a substituted amino group;

$X_3$ is a halogen atom, an amino group or -O-; $R_3$ is an optionally substituted lower alkyl, lower alkoxy or allyloxy group; and $R_4$ represents a
(2) selectively hydrolyzing the acridinium ester of nonhybridizing probes and those hybridizing probes which have a mismatch of at least one base in hybridization.

**12.** A method according to Claim 10 or 11 wherein HLA is a DR molecule in an antigen of class II.

**13.** A method according to Claim 10 or 11 wherein HLA is a DQ molecule in an antigen of class II.

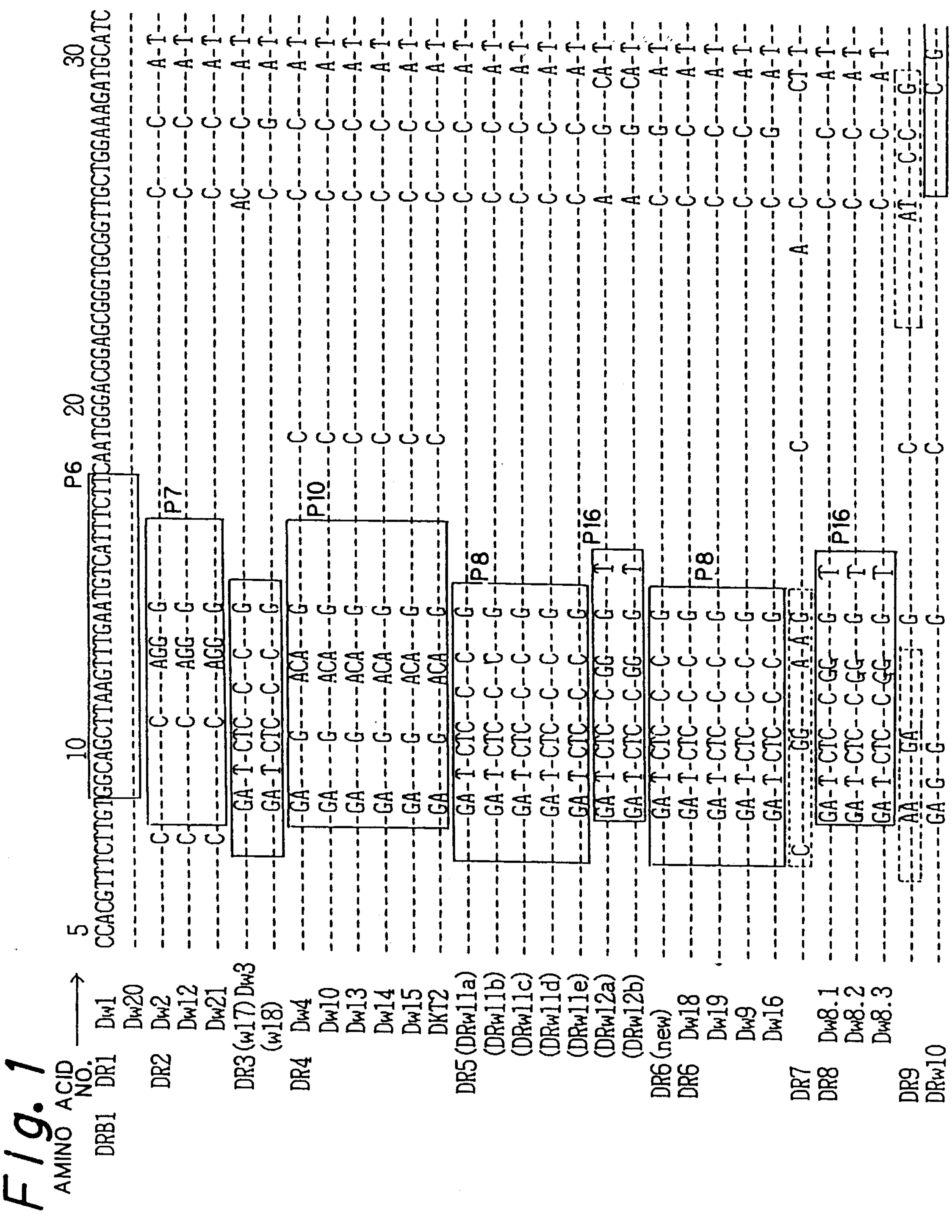
AMINO ACID NO.
5
10
20
30
CCACGTTTCTTGTGGCAGCTTAAGTTTGAATGTCATTTCTTCAATGGGACGGAGCGGGTGCGGTTGCTGGAAAGATGCATC
P6
P7
P10
P8
P16
DRB1
DR1 Dw1
Dw20
DR2 Dw2
Dw12
Dw21
DR3 (w17) Dw3
(w18)
DR4 Dw4
Dw10
Dw13
Dw14
Dw15
DKT2
DR5 (DRw11a)
(DRw11b)
(DRw11c)
(DRw11d)
(DRw11e)
(DRw12a)
(DRw12b)
DR6 (new)
DR6 Dw18
Dw19
Dw9
Dw16
DR7
DR8 Dw8.1
Dw8.2
Dw8.3
DR9
DRw10

Fig. 1

Fig. 2

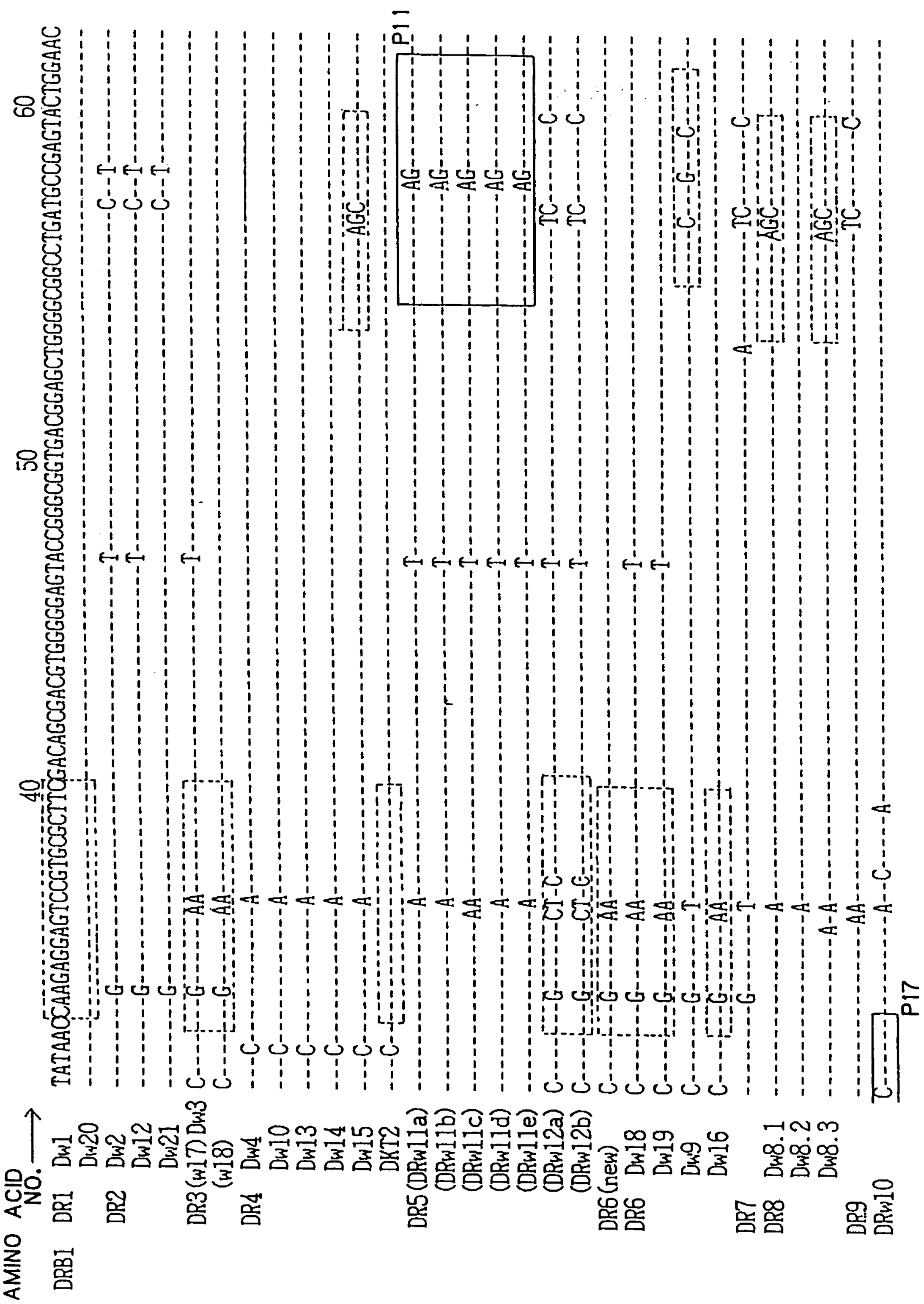
AMINO ACID NO.
DRB1
TATAACCAAGGAGTCCGTGCGCTTCGACAGCGACGTGGGGGAGTACCGGGCGGTGACGGAGCTGGGGCGGCCTGATGCCGAGTACTGGAAC
40
50
60
P11
P17
DR1 Dw1
Dw20
DR2 Dw2
Dw12
Dw21
DR3 (w17) Dw3
(w18)
DR4 Dw4
Dw10
Dw13
Dw14
Dw15
DKT2
DR5 (DRw11a)
(DRw11b)
(DRw11c)
(DRw11d)
(DRw11e)
(DRw12a)
(DRw12b)
DR6 (new)
DR6 Dw18
Dw19
Dw9
Dw16
DR7
DR8 Dw8.1
Dw8.2
Dw8.3
DR9
DRw10

Fig. 3

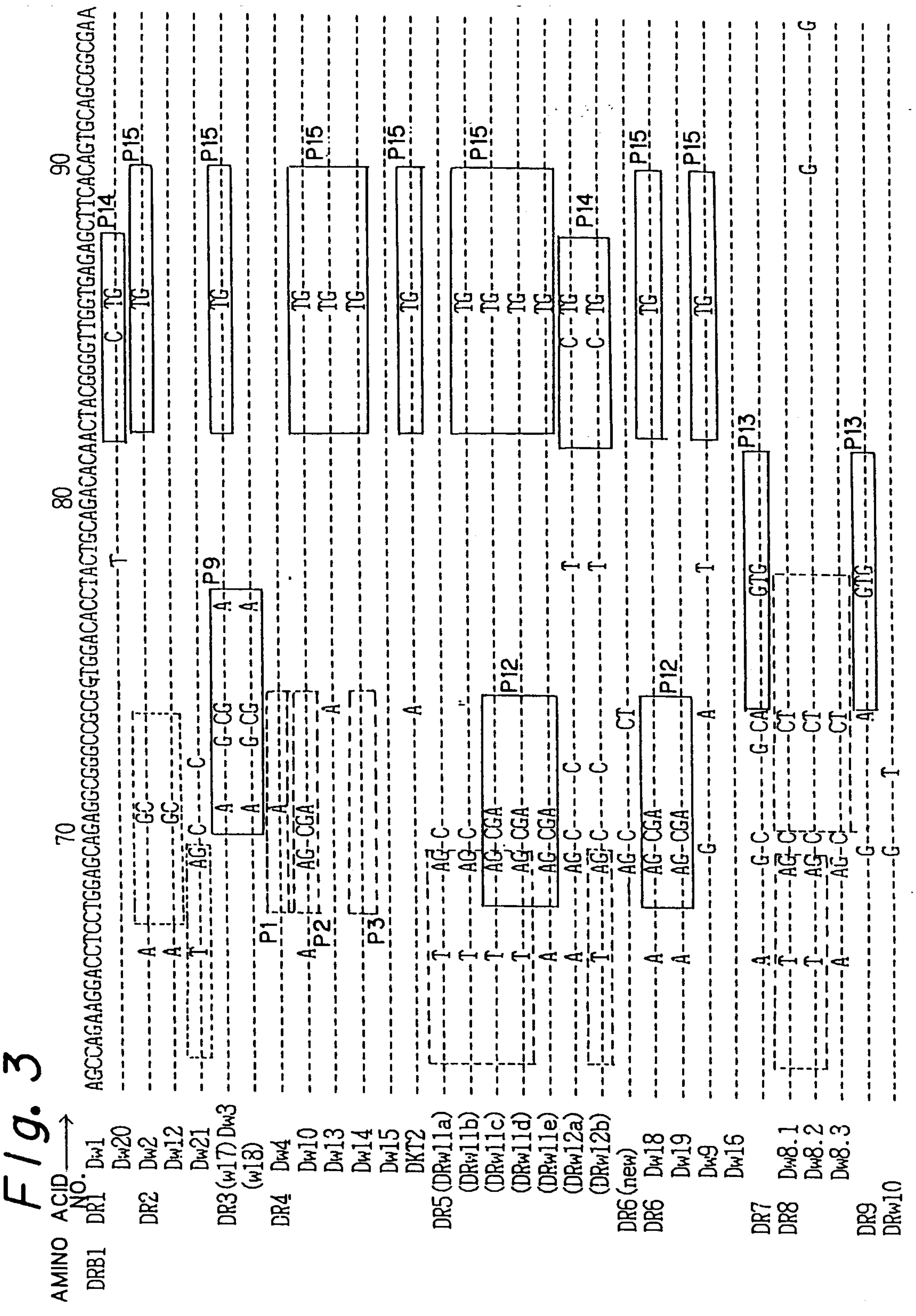
AMINO ACID NO.
DRB1
70
80
90
AGCCAGAAGGACCTCCTGGAGCAGAGGCGGGCCGCGGTGGACACCTACTGCAGACACAACTACGGGGTTGGTGAGAGCTTCACAGTGCAGCGGCGAA
DR1 Dw1
Dw20
DR2 Dw2
Dw12
Dw21
DR3 (w17) Dw3
(w18)
DR4 Dw4
Dw10
Dw13
Dw14
Dw15
DKT2
DR5 (DRw11a)
(DRw11b)
(DRw11c)
(DRw11d)
(DRw11e)
(DRw12a)
(DRw12b)
DR6 (new)
DR6 Dw18
Dw19
Dw9
Dw16
DR7
DR8 Dw8.1
Dw8.2
Dw8.3
DR9
DRw10
P1
P2
P3
P9
P12
P13
P14
P15

Fig. 4

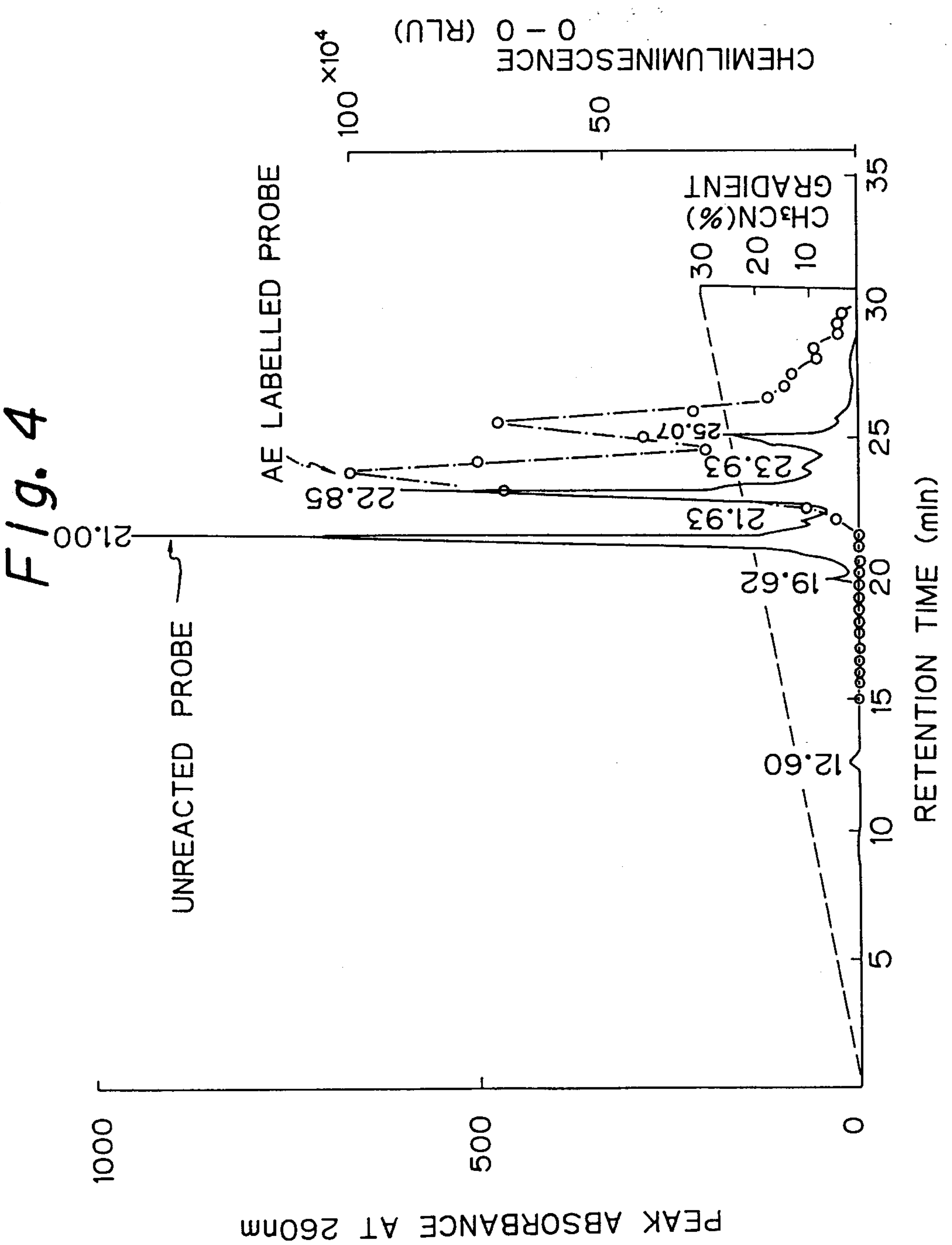
UNREACTED PROBE
AE LABELLED PROBE
21.00
22.85
21.93
23.93
25.07
19.62
12.60
RETENTION TIME (min)
PEAK ABSORBANCE AT 260nm
CHEMILUMINESCENCE
O – O (RLU)
$\times 10^4$
CH$_3$CN(%)
GRADIENT
0
5
10
15
20
25
30
35
500
1000
50
100

Fig. 5

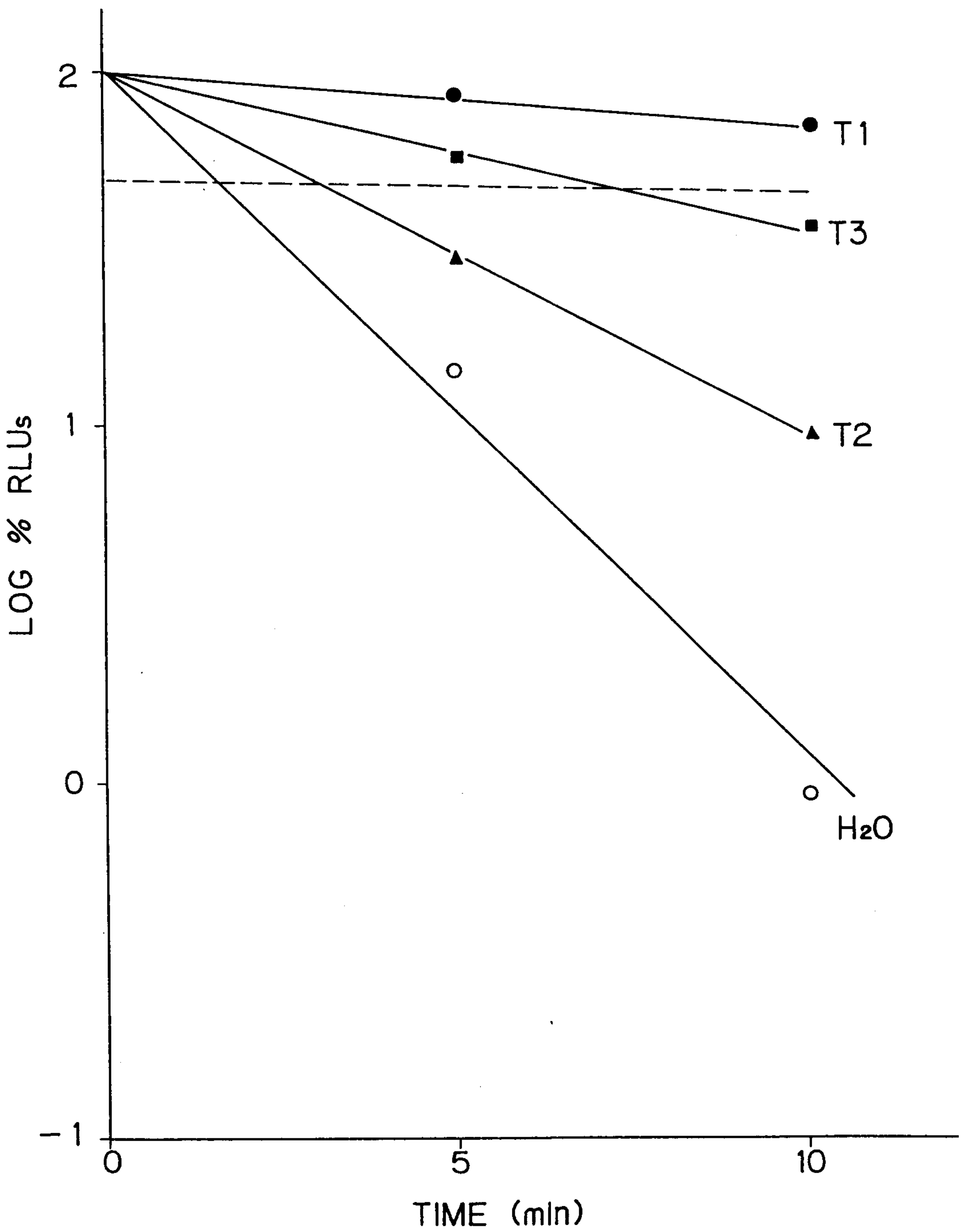

Fig. 6

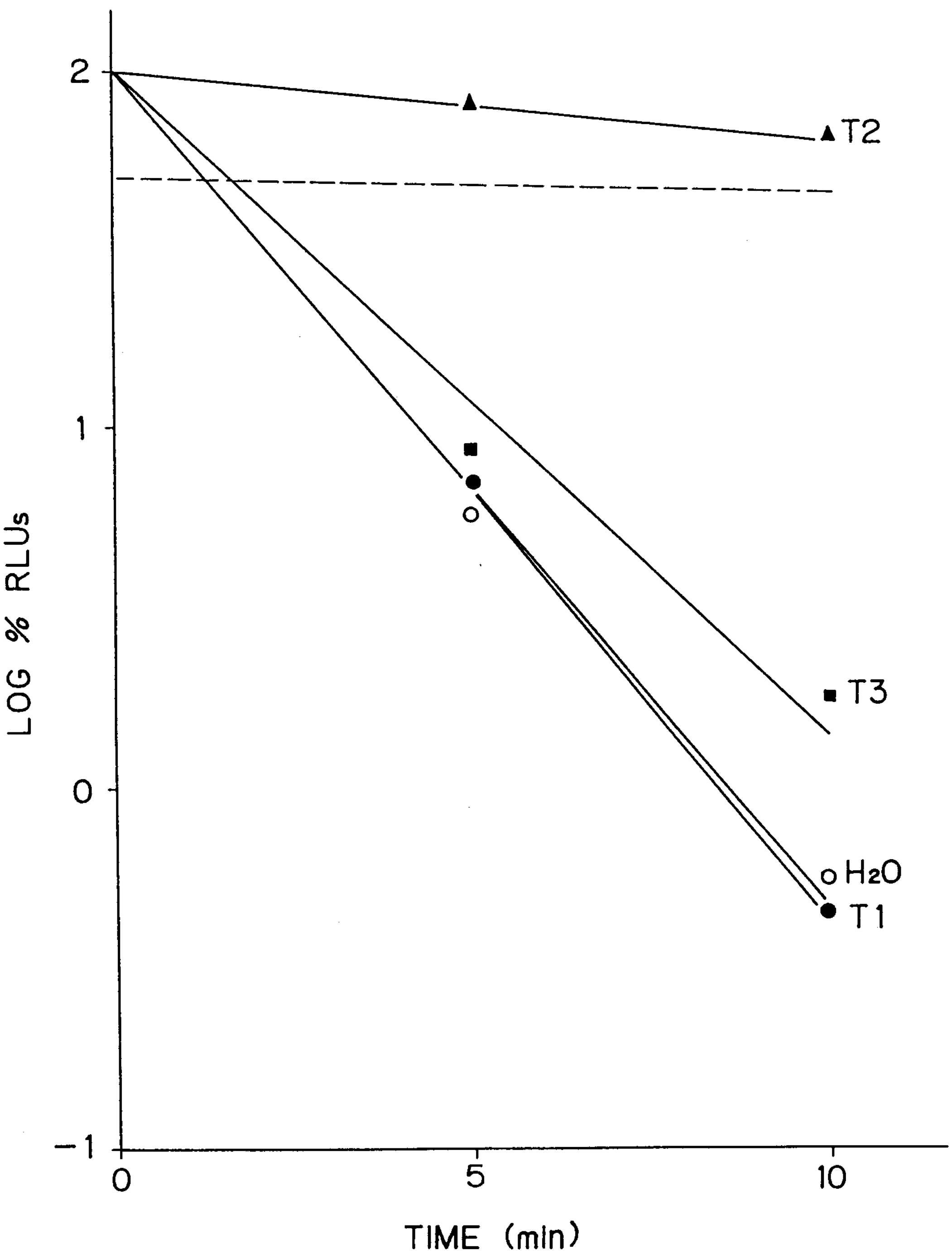

Fig. 7

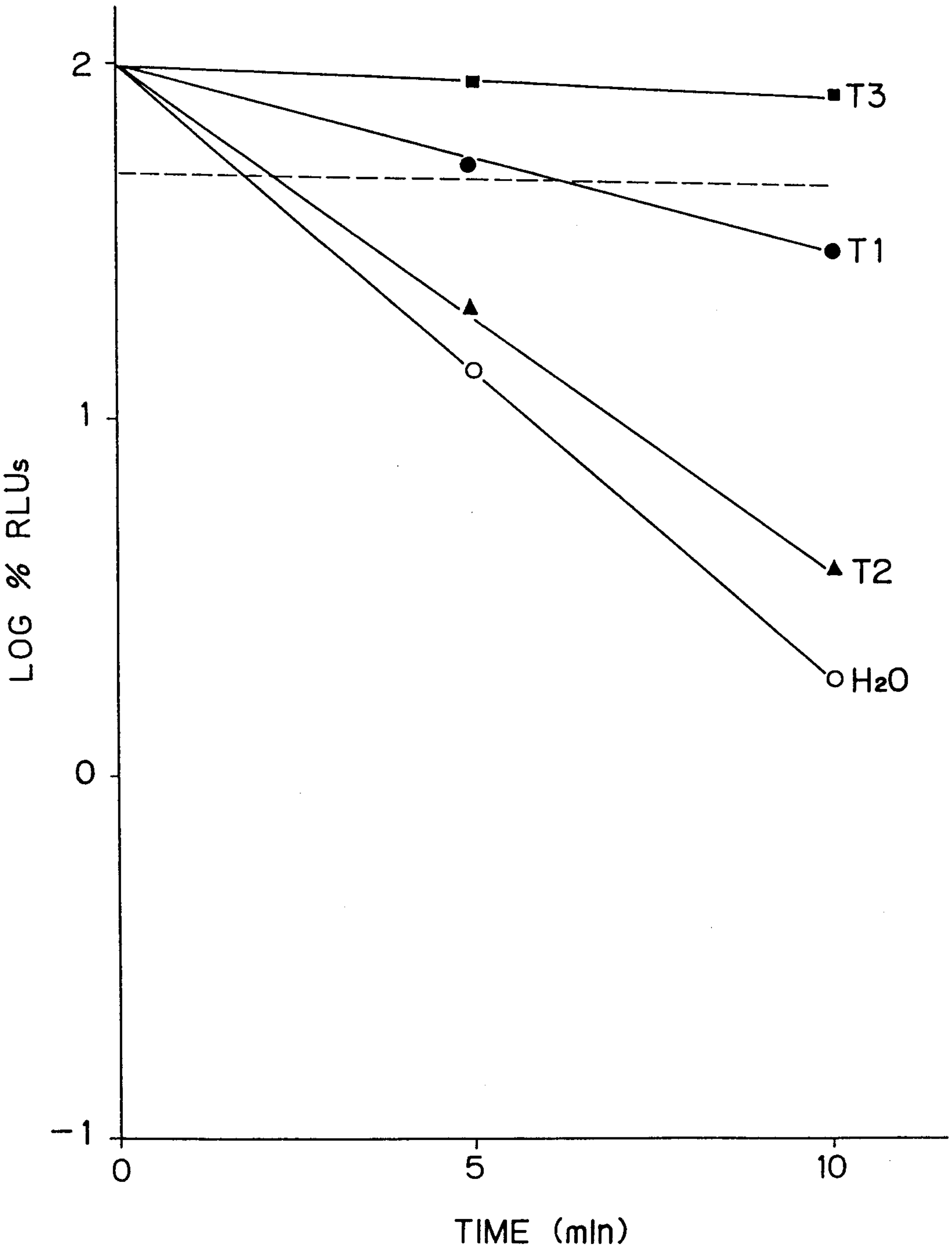

# Fig. 8

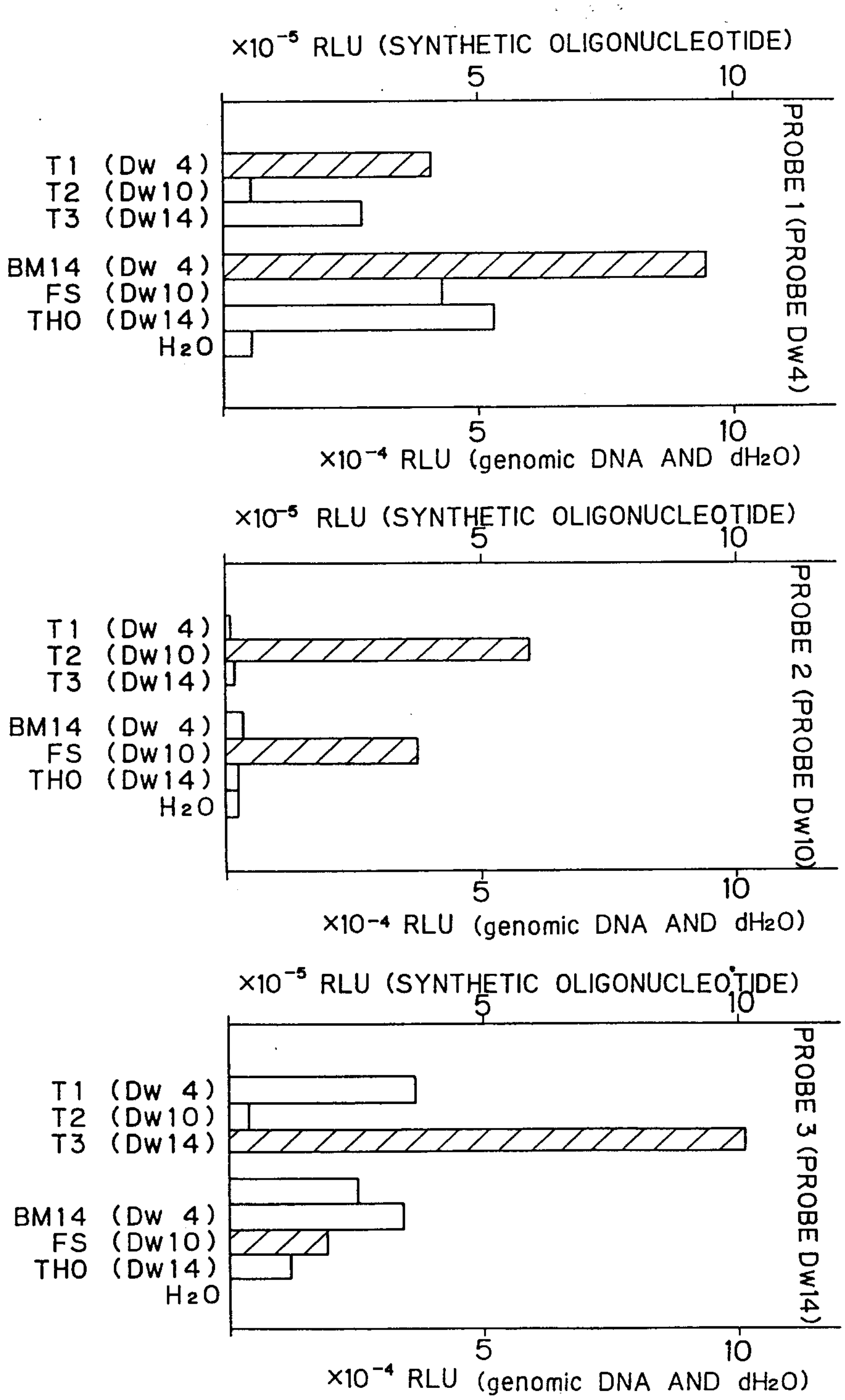
×10⁻⁵ RLU (SYNTHETIC OLIGONUCLEOTIDE)
5
10
T1 (Dw 4)
T2 (Dw10)
T3 (Dw14)
BM14 (Dw 4)
FS (Dw10)
THO (Dw14)
H2O
PROBE 1 (PROBE Dw4)
×10⁻⁴ RLU (genomic DNA AND dH2O)
×10⁻⁵ RLU (SYNTHETIC OLIGONUCLEOTIDE)
PROBE 2 (PROBE Dw10)
×10⁻⁴ RLU (genomic DNA AND dH2O)
×10⁻⁵ RLU (SYNTHETIC OLIGONUCLEOTIDE)
PROBE 3 (PROBE Dw14)
×10⁻⁴ RLU (genomic DNA AND dH2O)

*Fig. 9*

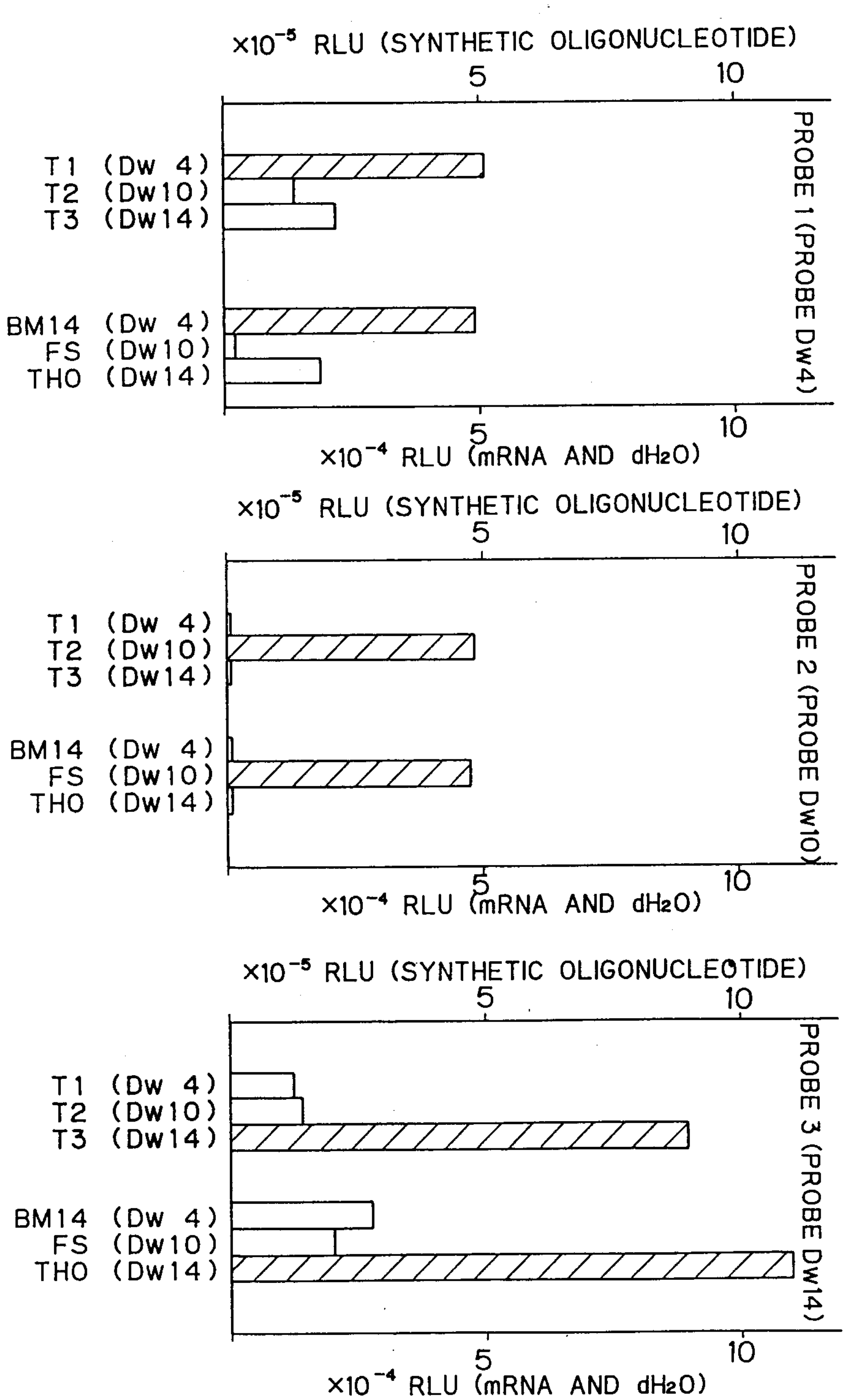
×10⁻⁵ RLU (SYNTHETIC OLIGONUCLEOTIDE)
5
10
T1 (Dw 4)
T2 (Dw10)
T3 (Dw14)
BM14 (Dw 4)
FS (Dw10)
THO (Dw14)
PROBE 1 (PROBE Dw4)
5
10
×10⁻⁴ RLU (mRNA AND dH2O)
×10⁻⁵ RLU (SYNTHETIC OLIGONUCLEOTIDE)
5
10
T1 (Dw 4)
T2 (Dw10)
T3 (Dw14)
BM14 (Dw 4)
FS (Dw10)
THO (Dw14)
PROBE 2 (PROBE Dw10)
5
10
×10⁻⁴ RLU (mRNA AND dH2O)
×10⁻⁵ RLU (SYNTHETIC OLIGONUCLEOTIDE)
5
10
T1 (Dw 4)
T2 (Dw10)
T3 (Dw14)
BM14 (Dw 4)
FS (Dw10)
THO (Dw14)
PROBE 3 (PROBE Dw14)
5
10
×10⁻⁴ RLU (mRNA AND dH2O)

# INTERNATIONAL SEARCH REPORT

International Application No PCT/JP91/00157

**I. CLASSIFICATION OF SUBJECT MATTER** (if several classification symbols apply, indicate all) [6]

According to International Patent Classification (IPC) or to both National Classification and IPC

Int. $Cl^5$ C12Q1/68, 1/04, G01N33/80//(C12Q1/04, C12R1:00)

**II. FIELDS SEARCHED**

Minimum Documentation Searched [7]

| Classification System | Classification Symbols |
|---|---|
| IPC | C12Q1/68, 1/04, G01N33/80 |

Documentation Searched other than Minimum Documentation to the Extent that such Documents are Included in the Fields Searched [8]

JICST Data Base

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | J. Biolumin. Chemilumin., Vol. 4, No. 1, 1989, Septak M. "Acridinium ester-labelled DNA oligonucleotide probes" p. 351-356 | 1-13 |
| A | Med. Immunol., Vol. 16, No. 6, 1988, Noboru Ujyo, Masahiro Maeda, Masaho Ota, Hidetoshi Inoko "HLA-DNA typing by the method of primer-directed enzymatic amplification" p. 949-956 | 1-13 |
| T | Clinical Pathology, No. June extra edition, 1990, Yukio Matsuoka, Kiyoshige Wakabayashi "Application of non-RI Marker DNA Probe to Examination" p. 82-91 | 1-13 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 3, 1991 (03. 04. 91) | April 15, 1991 (15. 04. 91) |
| **International Searching Authority** | **Signature of Authorized Officer** |
| Japanese Patent Office | |